# EUROPEAN PATENT APPLICATION

(11) **EP 1 045 037 A1**
(43) Date of publication of application: **18.10.2000**
(21) Application number: 99106213.4
(22) Date of filing: 12.04.1999
(51) Int. Cl.: C12Q 1/68, C12N 15/10

(54) **Plant cell identification system**

(71) Applicant: DISCOVERY BIOTECH., INC., Shin-Ying City, Tainan (TW); Kao, Chien-Yuan, Taipei (TW)
(72) Inventor: Kao, Chien-Yuan, Taipei (TW)
(74) Representative: Reinhard - Skuhra - Weise & Partner

(57) **Abstract**

The invention relates to a method of genetically marking a plant for identification by introducing into a plant genome a DNA sequence having specific information content. In some embodiments, the DNA sequence is less than 100 base pairs and is unique in the plant genome. In other embodiments, the DNA sequence does not contain a sequence which affects plant cell transcription or encodes a functional protein.

### Annex to the application documents - subsequently filed sequences listing

SEQUENCE LISTING

<110> Discovery Biotech., Inc.
      Kao, Chien-Yuan

<120> PLANT CELL IDENTIFICATION SYSTEM

<130> 10612/002EP1

<140> EP 99106213.4
<141> 1999-04-12

<160> 7

<170> FastSEQ for Windows Version 3.0

<210> 1
<211> 10
<212> DNA
<213> Artificial Sequence

<220>
<223> misc_feature

<400> 1
aaaaaaaaaa

<210> 2
<211> 10
<212> DNA
<213> Artificial Sequence

<220>
<223> misc_feature

<400> 2
cccccccccc

<210> 3
<211> 10
<212> DNA
<213> Artificial Sequence

<220>
<223> misc_feature

<400> 3
gggggggggg

<210> 4
<211> 10
<212> DNA
<213> Artificial Sequence

<220>
<223> misc_feature

<400> 4
tttttttttt

<210> 5
<211> 10
<212> DNA
<213> Artificial Sequence

<220>
<223> misc_feature

<400> 5
acacacacac

<210> 6
<211> 56
<212> DNA
<213> Artificial Sequence

<220>
<223> misc_feature

<400> 6
aaaaaaaaaa aaaccccaaa gtaaacccca aagtaaaccc caaagtaaaa aaaaaa

<210> 7
<211> 12
<212> DNA
<213> Artificial Sequence

<220>
<223> misc_feature

<400> 7
aaaccctaaa gt

## Description

### Background of the Invention

The development of various strains of plants having unique and commercially valuable properties has been an important arm of the agricultural sciences, even before the advent of modern biotechnology. Many such plants or cells thereof are proprietary, either through patent rights or as trade secrets. Thus, plant owners have an interest in tracking and identifying the distribution of their proprietary plants, some of which may have fallen into the hands of patent infringers or individuals who have misappropriated trade secrets.

Current methods of genetically identifying an organism such as a plant include detecting restriction fragment length polymorphisms (RFLP). RFLP analysis relies on the differences and similarities in the endogenous genomic sequence in one plant when compared to the endogenous genomic sequence in another plant.

### Summary of the Invention

The invention relates to a new method of registering a plant or plant cell by introducing predetermined DNA sequences into a plant genome. These DNA sequences can be readily detected or retrieved by standard procedures such as Southern blotting or polymerase chain reaction (PCR).

Accordingly, the invention features a method of genetically marking a plant for identification by introducing into a plant genome a DNA sequence of 100 base pairs or less, the DNA sequence being unique in the genome. The DNA sequence can be 80 base pairs or less (e.g., 60 or 40 base pairs or less), or at least 10 base pairs (e.g., at least 30, 50, or 70 base pairs) without exceeding the upper limit of 100 base pairs. The DNA sequence can include (1) a first sequence of 10 base pairs; (2) at least two direct repeats of an identification sequence of 6 or more base pairs; and (3) a second sequence of 10 base pairs. Alternatively, the DNA sequence can include (1) a first sequence of 10 base pairs; (2) an intervening sequence of 6 or more base pairs; and (3) a second sequence of 10 base pairs, where the first sequence and the second sequence are identical. Direct repeats refer to copies of a single sequence, all in the same orientation, along one strand of a doublestranded DNA molecule. By an identical sequence is meant the same sequence in the same 5' to 3' orientation.

The invention also includes a method of genetically marking a plant for identification by introducing a DNA sequence into a plant genome, the DNA sequence being unique in the genome and including, in linear order along the DNA sequence, (1) a first sequence of 10 base pairs; (2) at least two direct repeats of an identification sequence of 6 or more base pairs; and (3) a second sequence of 10 base pairs, where the first sequence, the second sequence, and the identification sequence are free of a sequence that alters transcription or encodes a functional protein. The DNA sequence optionally includes a selectable marker gene.

In addition, the invention features a method of genetically marking a plant for identification by introducing a DNA sequence into a plant genome, the DNA sequence being unique in the genome and free of a sequence that alters transcription or encodes a functional protein. The DNA sequence can include (1) a first sequence of 10 base pairs; (2) at least two direct repeats of an identification sequence of 6 or more base pairs; and (3) a second sequence of 10 base pairs. Alternatively, the DNA sequence can include (1) a first sequence of 10 base pairs; (2) an intervening sequence of 6 or more base pairs; and (3) a second sequence of 10 base pairs, where the first sequence and the second sequence are identical.

Further included in the invention is a method of genetically marking a plant for identification by introducing a DNA sequence into a plant genome, the DNA sequence being unique in the genome and including, in linear order along the DNA sequence, (1) a first sequence of 10 base pairs; (2) an intervening sequence of 6 or more base pairs; and (3) a second sequence of 10 base pairs, wherein the first sequence, the second sequence, and the identification sequence are free of a sequence that alters transcription or encodes a functional protein, where the first sequence and the second sequence are identical. The DNA sequence can optionally include a selectable marker gene.

The DNA sequences used in the methods of the invention can be introduced into the plant genome by any standard procedure known in the art, including via *Agrobacterium tumefaciens* or particle bombardment.

The selectable marker genes optionally used in the methods of the invention is any gene whose activity facilitates the selection of plant cells containing the introduced DNA sequence.

A functional protein is a polypeptide that has a known biochemical activity apart from its structural features (e.g., molecular weight, pKa, isoelectric point, and the like). Thus, a polypeptide exhibiting a molecular weight of 20 kDa on a gel without more is not a functional protein.

The methods of the invention provide a new means of identifying plants by introducing a unique foreign DNA sequence into the plant genome. In some embodiments, the DNA sequence does not contain a sequence which affects plant gene expression or encodes a functional protein. Such a DNA sequence has a variety of advantages. For example, the foreign DNA sequence does not affect the biology of the plant in any way, thereby alleviating governmental and societal fears that recombinant plants having biological activities different from a wild type plant are dangerous to humans or the environment. It is expected, therefore, that plants approved for human consumption or cultivation in the field can be genetically marked without regulatory hurdles.

Other features or advantages of the present invention will be apparent from the following drawings and detailed description.

### Brief Description of the Drawings

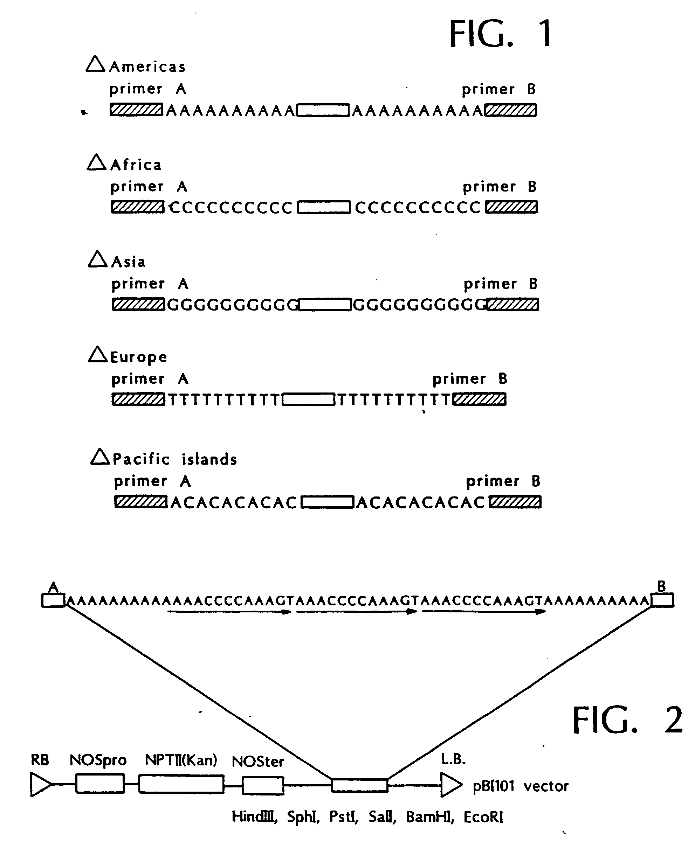

Fig. 1 is a schematic representation of several DNA sequences used in the methods of the invention to identify the geographic origin of a plant. Ten base pair direct repeats represent the geographic origin of the plant, e.g., from the Americas (AAAAAAAAAA; SEQ ID NO:1), Africa (CCCCCCCCCC; SEQ ID NO:2), Asia (GGGGGGGGGG; SEQ ID NO:3), Europe (TTTTTTTTTT; SEQ ID NO:4), or pacific islands (ACACACACAC; SEQ ID NO:5). Each box between the direct repeats represents additional sequence information about the nature and origin of the plant.

Fig. 2 is a schematic representation of a specific 100 base pair DNA sequence, excluding the primer sequences, (AAAAAAAAAAAAACCCCAAAGTAAACCCCAAAGTAAACCCCAAAGTAAAAAAAAAA; SEQ ID NO:6) used to mark a plant originating from the Americas.

### Detailed Description

The invention is based on a new means of genetically marking a plant or plant cell by introducing an artificial DNA sequence into the plant cell genome.

Contemplated within the scope of the invention is the use of short (100 base pairs or less). The short sequence can be divided into four sections to specify the plant owner (e.g., a corporation), geographic origin (e.g., Europe), country origin (e.g., Germany), and other plant characteristics (e.g., tomato).

Without further elaboration, it is believed that one skilled in the art can, based on the above disclosure and the description below, utilize the present invention to its fullest extent. The following description is to be construed as merely illustrative of how one skilled in the art can practice the invention and are not limitative of the remainder of the disclosure in any way. Any publications cited in this disclosure are hereby incorporated by reference.

### One Hundred Base Pair Registration Sequence

A short piece of DNA of 100 base pairs or less and its complement can be synthesized using standard oligonucleotide synthesis techniques. The pieces can be modified to contain flanking sequences for cloning into plasmid vectors. One type of DNA sequences useful in the methods of the invention is illustrated in Fig 1.

Referring to Fig. 1, a set of DNA sequences, each marking plants from a particular region of the world, are shown. Moving from 5' to 3', the 100 base pair DNA sequences include (1) a 22 base pair primer sequence A, (2) a 10 base pair sequence representing the geographic origin of the plant (SEQ ID NOs:1-5), (3) a 36 base pair identification sequence as described below, (4) a direct repeat of the 10 base pair sequence, and (5) a 22 base pair primer sequence B. The identification sequence is three direct repeats of a 12 nucleotide sequence, which is composed of, in linear order, 3 nucleotides to specify the plant's country of origin, 4 nucleotides to specify the company owner, and 5 nucleotides to specify the nature of the plant. This "3 + 4 + 5" ID sequence is repeated three times without intervening sequences between the repeats.

In general, the identification sequence contains information that unambiguously specifies the marked plant. In the above example, the identification sequence includes sequences marking the plant's country of origin, the plant owner, and the nature of variety of the plant.

To introduce these sequences into a plasmid vector (e.g., an *Agrobacterium tumefaciens* binary vector, such as the standard pBI101 vector with the kanamycin selectable marker removed), the ID sequence can be chemically or enzymatically introduced into a vector. The complete 100 base pair sequence can be introduced into the appropriate region of the vector by standard cloning techniques (Sambrook et al., eds., *Molecular Cloning: A Laboratory Manual. 2nd, ed., Cold Spring Harbor Laboratory,* Cold Spring Harbor Laboratory Press, Cold Spring Harbor, NY, 1989).

The pBI101 containing the sequence to be introduced into the plant genome can be used to transform the common *A. tumefaciens* strain LBA4404 by electroporation or freeze/thaw. The transformed bacteria are then used to infect target plant cells. After cloning and culturing the plant cells, individual clones (typically at least 10 clones) containing the 100 base pair sequence are confirmed by isolating the genomic DNA from the clones and performing PCR using primer A and primer B. The PCR products, if any, are optionally sequenced. Confirmed clones are then differentiated into mature plants.

A secondary confirmation of integrated identification sequences can be done by isolating genomic DNA from the first shoot, seedling, or other tissues, and performing PCR using primers A and B. Again, the PCR products can also be sequenced. At this time, the phenotype and ploidy of the plant should also be confirmed.

Additional details regarding the plasmids and bacterial strains discussed above can be found in Kado, Genet Eng 20:1-24, 1998; McCormac et al., Mol Biotechnol 9:155-159, 1998; Guerrero et al., Plant Mol Biol 21:929-935, 1993; and references cited therein.

### Inclusion of a Selectable Marker

To facilitate the isolation of plant cell clones containing the registration sequence, the 100 base pair registration sequence described above can be cloned into the wild type pBI101 vector as shown in Fig. 2. The plasmid is then introduced into plant cells by gold particle bombardment (see, e.g., Wakita et al., Genes Genet Syst 73:219-226, 1998; Sawasaki et al., Gene 218:27-35, 1998; and references cited therein). Briefly, 37 µl of a 40 mg/ml gold stock solution (1.6 µm diameter; BioRad) is mixed sequentially with 25 µl water and 2 µg plasmid DNA in a 250 µl Eppendorf tube. The tube is vortexed before and after each addition. 20 µl of 100 nM free base spermidine and 50 µl of 2.5 M CaCl₂ are placed in separate drops on the side of the tube to avoid premature mixing of either solution with the DNA/gold solution. The tube is then mixed by vortexing for 10 second to attach the DNA to the gold particles, which are precipitated. The tube is centrifuged for 5 seconds, and the supernatant is removed. 100 µl of 100% ethanol are added, and the tube is place on ice. A 5 µl volume of sonicated gold/DNA mixture is then used for each bombardment shot.

The bombarded cells are then grown in media containing sufficient kanamycin to select for plant cells containing the 100 base pair sequence. Each grouping or piece of kanamycin-resistant tissue (typically at least 10 clones) is considered an independent transformation event. The transformed tissue is then differentiated into adult plants. Confirmation of the presence of the registration sequence is performed as described above.

### Registration Number System

The integrated registration sequence can be PCR amplified from any registered plant and sequenced. The resulting sequence can be used to assign a registration number to the plant. For example, the "3 + 4 + 5" ID sequence is converted into a number as follows. For each base, an A = 1, a C = 2, a G = 3, and a T = 4. The 12 base pair direct repeat sequence of AAACCCTAAAGT (SEQ ID NO:7; see Fig. 2) is then converted into the string of digits 111222411134. Note that the sequence CCCT and the corresponding digits 2224 represent the plant's owner (see above). The digits derived from the 12 base pair sequence are added together to yield a sum of 23. Under this system, the plant would therefore contain a genetically embedded registration number of 2224.23.

When the embedded registration sequence is free of a sequence affecting plant transcription or a sequence encoding a functional protein, the registration sequence remains hidden to everyone except the owners who have kept secret the primer A sequence, the primer B sequence, or both. This is so because, biologically and biochemically, these marked plants are no different from the original unmarked plants.

### Other Embodiments

It is to be understood that while the invention has been described in conjunction with the detailed description thereof, the foregoing description is intended to illustrate and not limit the scope of the invention, which is defined by the scope of the appended claims. Other aspects, advantages, and modifications are within the scope of this invention.

For example, additional layers of encryption can be used with the methods of the invention to safeguard the transparency of the embedded genetic marker.

## Claims

1. A method of genetically marking a plant for identification, the method comprising introducing into a plant genome a DNA sequence consisting of 100 base pairs or less, the DNA sequence being unique in the genome.

2. The method of claim 1, wherein the DNA sequence consists of 80 base pairs or less.

3. The method of claim 2, wherein the DNA sequence consists of 60 base pairs or less.

4. The method of claim 3, wherein the DNA sequence consists of 40 base pairs or less.

5. The method of claim 1, wherein the DNA sequence comprises (1) a first sequence of 10 base pairs; (2) at least two direct repeats of an identification sequence of 6 or more base pairs; and (3) a second sequence of 10 base pairs.

6. The method of claim 1, wherein the DNA sequence comprises (1) a first sequence of 10 base pairs; (2) an intervening sequence of 6 or more base pairs; and (3) a second sequence of 10 base pairs, wherein the first sequence and the second sequence are identical.

7. The method of claim 1, wherein the DNA sequence is introduced into the plant genome by *Agrobacterium tumefaciens.*

8. The method of claim 1, wherein the DNA sequence consists of at least 10 base pairs.

9. The method of claim 8, wherein the DNA sequence consists of at least 30 base pairs.

10. The method of claim 9, wherein the DNA sequence consists of at least 50 base pairs.

11. The method of claim 1, wherein the DNA sequence consists of at least 70 base pairs.

12. A method of genetically marking a plant for identification, the method comprising introducing a DNA sequence into a plant genome, the DNA sequence being unique in the genome and comprising, in linear order along the DNA sequence, (1) a first sequence of 10 base pairs; (2) at least two direct repeats of an identification sequence of 6 or more base pairs; and (3) a second sequence of 10 base pairs, wherein the first sequence, the second sequence, and the identification sequence are free of a sequence that alters transcription or encodes a functional protein.

13. The method of claim 11, wherein the DNA sequence further comprises a selectable marker gene.

14. The method of claim 11, wherein the DNA sequence is introduced by *Agrobacterium tumefaciens.*

15. A method of genetically marking a plant for identification, the method comprising introducing a DNA sequence into a plant genome, the DNA sequence being unique in the genome and free of a sequence that alters transcription or encodes a functional protein.

16. The method of claim 15, wherein the DNA sequence comprises (1) a first sequence of 10 base pairs; (2) at least two direct repeats of an identification sequence of 6 or more base pairs; and (3) a second sequence of 10 base pairs.

17. The method of claim 15, wherein the DNA sequence comprises (1) a first sequence of 10 base pairs; (2) an intervening sequence of 6 or more base pairs; and (3) a second sequence of 10 base pairs, wherein the first sequence and the second sequence are identical.

18. The method of claim 15, wherein the DNA sequence is introduced by *Agrobacterium tumefaciens.*

19. A method of genetically marking a plant for identification, the method comprising introducing a DNA sequence into a plant genome, the DNA sequence being unique in the genome and comprising, in linear order along the DNA sequence, (1) a first sequence of 10 base pairs; (2) an intervening sequence of 6 or more base pairs; and (3) a second sequence of 10 base pairs, wherein the first sequence, the second sequence, and the intervening sequence are free of a sequence that alters transcription or encodes a functional protein, and wherein the first sequence and the second sequence are identical.

20. The method of claim 19, wherein the DNA sequence further comprises a selectable marker gene.
